# EUROPÄISCHE PATENTSCHRIFT

(11) **EP 2 893 945 B1**
(45) Veröffentlichungstag und Bekanntmachung des Hinweises auf die Patenterteilung: **09.08.2017**
(21) Anmeldenummer: 15150353.9
(22) Anmeldetag: 07.01.2015
(51) Int. Cl.: A61M 1/14

(54) **Dialysemaschine mit Leckageerkennung sowie ein Verfahren zur Erkennung von Leckagen in Dialysierflüssigkeitssystemen**
Dialysis machine with leakage detection and a method of detecting leaks of dialysis fluid systems
Machine de dialyse à détection de fuites et procédé de détection de fuites dans des systèmes de liquides de dialyse

(30) Priorität: 10.01.2014 DE 102014100260
(43) Veröffentlichungstag der Anmeldung: 15.07.2015
(73) Patentinhaber: B. Braun Avitum AG, 34212 Melsungen (DE)
(72) Erfinder: Ritter, Kai-Uwe, 91126 Rednitzhembach (DE); Schulz, Oliver, 34253 Lohfelden (DE)
(74) Vertreter: Winter, Brandl, Fürniss, Hübner, Röss, Kaiser, Polte - Partnerschaft mbB

(56) Entgegenhaltungen:
- US-A1- 2005 101 901
- US-A1- 2009 012 454
- US-A1- 2011 272 337

## Beschreibung

Die Erfindung betrifft eine Dialysemaschine mit Leckageerkennung sowie ein Verfahren zur Erkennung von Leckagen im Dialysierflüssigkeits- oder Dialysatsystem einer Dialysemaschine.

Leckagen, die im Dialysierflüssigkeitssystem einer Dialysemaschine, insbesondere im Bilanzierungskreis einer Dialysemaschine, auftreten, sind kritisch, da aus dem System ausgetretene Dialysierflüssigkeit in der Gesamtbilanz fehlt und aufgrund des Bilanzierungsprinzips dem Patienten eine entsprechende Menge an Flüssigkeit entzogen wird. Ein solcher Flüssigkeitsentzug kann je nach Gewicht und/oder Zustand des Patienten bereits in kleinen Mengen kritisch sein.

Es ist derzeit bekannt, zur Erkennung von Leckagen im Dialysierflüssigkeitssystem einen einmaligen Drucktest oder zyklische Drucktests durchzuführen. In der Regel finden diese vor einer Behandlung statt. Es ist dabei von Nachteil, dass Leckagen, die erst während einer Behandlung auftreten, nicht oder erst bei der Vorbereitung einer nachfolgenden Behandlung erkannt werden können. Eine Durchführung von zyklischen Drucktests während einer Behandlung führt mit Nachteil zu Verzögerungen des Therapieablaufs und damit zu einer effektiven Verkürzung der Therapiedauer, was durch längere Behandlungszeiten je Patient auszugleichen ist. Generell ist die Genauigkeit bei Drucktests mit Nachteil abhängig von der Höhe des Drucks sowie der Druckhaltezeit, was die Verlässlichkeit der ermittelten Werte in Frage stellen kann.

Aus dem Stand der Technik sind des Weiteren Lösungen bekannt, bei denen Leckageflüssigkeit in einem geeigneten Behälter der Dialysemaschine gesammelt wird. Über einen Füllstandssensor oder Ähnliches wird der Flüssigkeitspegel in dem Behälter erfasst. Bei diesen Lösungen ist es von Nachteil, dass sich neben aus dem System austretender Dialysierflüssigkeit in dem Behälter weitere Flüssigkeit ansammeln kann, wie zum Beispiel Kondenswasser, was zu Verfälschungen bei der Bestimmung der aus dem System ausgetretenen Menge an Dialysierflüssigkeit führt. Auch im umgekehrten Fall, dass nicht die gesamte aus dem System aufgrund von Leckage ausgetretene Dialysierflüssigkeitsmenge in dem Behälter gesammelt wird, zum Beispiel da ein bestimmter Anteil davon verdunstet, kommt es bei der Bestimmung der aus dem System ausgetretenen Menge zu Verfälschungen. Die US 2011/0272337 A1 offenbart ein tragbares Dialysegerät. Dies weist einen Leckdetektor auf. Ein Feuchtigkeitssensor überwacht die Feuchtigkeit im Gehäuse zum Beispiel an einem Auslass des Gerätes.

Grundsätzlich ist eine Leckageerkennung nach dem Stand der Technik mit einer gewissen Unsicherheit und Ungenauigkeit behaftet. Es besteht die Gefahr, dass geringe Leckagen, die je nach Patient bereits gravierende Auswirkungen haben können, noch nicht oder nur ungenau erkannt werden. Verlässlich funktionieren Systeme nach dem Stand der Technik in der Regel erst bei höheren Leckageraten. Teilweise bewirken bekannte Leckageerkennungen Zeitverlust bei und Qualitätsminderung von Dialysebehandlungen.

Ausgehend von dem vorstehend beschriebenen Stand der Technik liegt der vorliegenden Erfindung die Aufgabe zugrunde, die zuvor angeführten Nachteile zu beseitigen, insbesondere eine Möglichkeit zur Leckageerkennung im Dialysierflüssigkeitssystem einer Dialysemaschine zu schaffen, die auch schon bei kleinen Leckagemengen vergleichsweise sicher, genau und zuverlässig arbeitet, mit der eine fortwährende Überwachung des Systems hinsichtlich Leckage, insbesondere auch während einer laufenden Behandlung im Wesentlichen ohne deren Beeinträchtigung, möglich ist, die keine wesentliche Verlängerung oder Unterbrechung der Verhandlung bewirkt oder erforderlich macht und vergleichsweise kostengünstig ist.

Die vorstehend genannte Aufgabe sowie weitere Ziele der vorliegenden Erfindung werden durch eine Dialysemaschine mit den Merkmalen des Anspruchs 1 sowie durch ein Verfahren mit den Verfahrensschritten des Anspruchs 6 gelöst. Vorteilhafte Weiterbildungen der Erfindung sind Gegenstand der Unteransprüche.

Nach der Erfindung wird diese Aufgabe gemäß einem ersten Aspekt gelöst durch eine Dialysemaschine mit einem Gehäuse, das wenigstens eine erste Belüftungsöffnung und wenigstens eine zweite Belüftungsöffnung aufweist, wobei das Gehäuse mit Ausnahme der Belüftungsöffnungen gegenüber der Umgebung hermetisch dicht ausgebildet ist und in dem Gehäuse zumindest ein hinsichtlich Leckage zu erfassender/zu testender Teil eines Dialysierflüssigkeitssystems aufgenommen ist, vorzugsweise das gesamte Dialysierflüssigkeitssystem, wobei wenigstens eine Lüftungseinrichtung vorgesehen ist, mittels der Luft von einer der Belüftungsöffnungen (Ansaugöffnung) durch das Gehäuse zur anderen (Ausblasöffnung) der Belüftungsöffnungen förderbar ist bzw. gefördert wird, wobei die Dialysemaschine des Weiteren wenigstens eine Meßeinrichtung aufweist, mittels der jeweils die Feuchtigkeit der über die Belüftungsöffnungen in das Gehäuse ein- und aus dem Gehäuse ausströmenden Luft bestimmbar ist bzw. bestimmt wird.

Die Aufgabe wird des Weiteren gemäß einem anderen Aspekt der Erfindung gelöst durch ein Verfahren zum Erfassen von Leckagen in einem Dialysierflüssigkeitssystem einer Dialysemaschine, bei dem ein Gehäuse, das wenigstens eine erste Belüftungsöffnung (Ansaugöffnung) und wenigstens eine zweite Belüftungsöffnung (Ausblasöffnung) aufweist und im Übrigen gegenüber der Umgebung hermetisch dicht (fluiddicht) ist, zumindest einen hinsichtlich Leckage zu erfassenden / zu überwachenden Teil des Dialysierflüssigkeitssystems enthält, vorzugsweise das gesamte Dialysierflüssigkeitssystem enthält, wobei das Gehäuse über die Belüftungsöffnungen belüftet wird, vorzugsweise ausschließlich über die Belüftungsöffnungen belüftet wird, so dass ein Eindringen von Luft und/oder Feuchtigkeit außer über die Belüftungsöffnungen verhindert werden kann, wobei Zustandsparameter, wie insbesondere Luftmasse oder Luftmassenstrom, Temperatur und/oder Feuchtigkeit etc., von in das Gehäuse zugeführter Zuluft sowie von aus dem Gehäuse abgeführter Abluft erfasst und vorzugsweise verglichen werden, und wobei aus der Differenz dieser Werte ein Feuchtigkeitseintrag in das Gehäuse basierend aus einer Leckage am Dialysierflüssigkeitssystem innerhalb des Gehäuses bestimmt wird.

Der in dem Gehäuse aufgenommene Teil des Dialysierflüssigkeitssystems der Maschine, der hinsichtlich Leckage zu erfassen ist, ist gegenüber der Umgebung hermetisch abgeschirmt, vorzugsweise mittels des Gehäuses. Er ist damit gegenüber externen Feuchtigkeitsquellen abgeschirmt. Ausnahmen sind das Dialysierflüssigkeitssystem der Maschine im Falle einer Leckage sowie Luft, die über die Belüftungsöffnungen in das Gehäuse gelangt. Anders ausgedrückt ist das Gehäuse konstruktiv so gestaltet, dass Luft nur über die oder eine Belüftungsöffnung in das Gehäuse eindringen kann. Die Feuchtigkeit oder der Feuchtigkeitseintrag von über die Belüftungsöffnungen (kontrolliert) in das Gehäuse eindringender Luft wird nach der Lehre der Erfindung erfasst und bilanziert. Auf diese Weise kann ein definierter und in sich geschlossener Luftweg gebildet sein oder werden, bei dem wenigstens ein (die Luftfeuchtigkeit repräsentierender oder auf die Luftfeuchtigkeit hinweisender) Parameter der das Gehäuse durchströmenden Luft, vorzugsweise der dem Gehäuseinneren zuströmenden Zuluft und/oder der aus dem Gehäuseinneren herausströmenden Abluft bestimmt werden kann, z.B. mittels eines Feuchtesensors. Das Gehäuse bildet somit eine Art Bilanzhülle, wobei sämtliche Feuchtigkeitsein- und -austräge in das bzw. aus dem Gehäuse heraus (sensorisch) erfassbar sind, mit Ausnahme von aus Leckagen des Dialysierflüssigkeitssystems stammender Feuchtigkeit, die jedoch indirekt über eine Bilanzierung bestimmbar ist.

Im Falle einer Leckage von Diaysierflüssigkeit aus dem (gesamten) Dialysierflüssigkeitssystem oder aus dessen hinsichtlich Leckage zu überwachendem Teil gelangt die ausgeleckte Dialysierflüssigkeit zwangsläufig in das Innere des Gehäuses. Hier verdunstet die ausgetretene Dialysierflüssigkeit, was zu einer Erhöhung der Luftfeuchtigkeit im Inneren des Gehäuse und damit des Abluftstroms aus dem Gehäuse heraus führt. Vorzugsweise sind Mittel vorgesehen, die eine rasche und vollständige Verdunstung von Leckageflüssigkeit bewirken, z.B. ein Vlies oder ein zusätzlicher Lüfter, der die gewünschte Verdunstung durch zusätzliche und gezielte Luftbewegung fördert. Aus der Differenz der Luftfeuchtigkeit anzeigenden oder darauf hinweisenden Parameter von Zu- und Abluft kann bestimmt werden, ob und ggf. in welcher Menge zusätzlich Feuchtigkeit in das Belüftungssystem, insbesondere in das Gehäuse, gelangt. Über Zuluft eingebrachte Feuchtigkeit wird dabei zwangsläufig erfasst und beachtet. Werden Parameter von Zu- und Abluft mit ausreichender Genauigkeit bestimmt, können mit Hilfe der vorliegenden Erfindung bereits kleinste Leckageraten ermittelt werden.

Eine Dialysemaschine weist grundsätzlich zwei Kreisläufe auf, den extrakorporalen Blutkreislauf sowie den Dialysierflüssigkeitskreislauf. Der extrakorporale Blutkreislauf und der Dialysierflüssigkeitskreislauf stehen über einen Dialysator in Verbindung. Aus dem Dialysierflüssigkeitskreislauf kann physiologische Flüssigkeit, insbesondere Dialysierflüssigkeit, dem extrakorporalen Blutkreislauf zugeführt werden, z.B. um überhöhte (ungewollte) Flüssigkeitsverluste des Patienten auszugleichen. Unter dem Dialysierflüssigkeitssystem im Sinne der vorliegenden Beschreibung ist jener Teil der Maschine zu verstehen, der physiologische Flüssigkeit, insbesondere Dialysierflüssigkeit, führt, insbesondere der Dialysierflüssigkeitskreislauf sowie von diesem abzweigende Bestandteile und Leitungen, die ihn mit dem extrakorporalen Blutkreislauf verbinden, oder Teile davon.

Bei einer bevorzugten Ausführungsform der Erfindung weist die wenigstens eine Lüftungseinrichtung einen Lüfter auf oder ist durch einen solchen ausgebildet. Dieser kann unter Umständen bei der Dialysemaschine ohnehin vorhanden sein und vorzugsweise einer parallelen oder gleichzeitigen Kühlung der Maschine dienen. Mit besonderem Vorteil kann das Gehäuse durch die Lüftungseinrichtung entlüftet werden, d.h. Luft mittels der Lüftungseinrichtung aus dem Gehäuse heraus, vorzugsweise in die Umgebung, gefördert werden. Dabei bildet sich im Gehäuse gegenüber der Umgebung ein Unterdruck, so dass die Wahrscheinlichkeit eines versehentlichen Luftaustritts außer über die Belüftungseinrichtung bzw. die Abluftöffnung, durch den eine Erfassung von in die Luft innerhalb des Gehäuses aufgrund einer Leckage eingebrachter Feuchtigkeit verfälscht oder verhindert werden könnte, verringert werden kann.

Es liegt im Bereich der Erfindung, ein Gehäuse mit nur einer Zuluftöffnung und nur einer Abluftöffnung zu verwenden. Das Gehäuse kann auch mehrere Zuluftöffnungen und/oder mehrere Abluftöffnungen aufweisen. Nach der Erfindung werden Parameter der Luftströmungen an allen Zu- bzw. Abluftöffnungen überwacht und erfasst.

Nach einer Ausführungsform der Erfindung wird wenigstens ein Parameter der das Gehäuse durchströmenden Luft, vorzugsweise des in das Gehäuse einströmenden Zuluftstroms und/oder des aus dem Gehäuse ausströmenden Abluftstroms, fortwährend erfasst. Es liegt außerdem im Bereich der Erfindung, einen oder jeden Parameter fortwährend oder zyklisch zu erfassen. Insbesondere können wenigstens ein Parameter oder alle Parameter in diskreten Messzyklen erfasst werden. Zwischen den einzelnen Messzyklen können zeitliche Zwischenräume von jeweils gleicher oder unterschiedlicher Länge liegen. Die Belüftung des Gehäuses während dieser zeitlichen Zwischenräume kann mit Vorteil nicht nur der Erfassung eines Parameters zur Leckageerkennung, sondern (insbesondere auch ausschließlich) der Kühlung des Gehäuseinnenraums und der Dialysemaschine dienen.

Nach der Erfindung können der Volumenstrom und/oder der Massestrom und/oder die Strömungsgeschwindigkeit der durch das Gehäuse geleiteten Luft konstant sein. Nach einer besonderen Form der Erfindung können diese Parameter der durch das Gehäuse geleiteten Luft jedoch variiert werden. Eine solche Variation kann im Falle von diskreten Messzyklen mit Vorteil zyklusspezifisch durchgeführt werden. Beispielsweise kann oder können nach der Erfindung der Volumenstrom bzw. der Massenstrom bzw. die Strömungsgeschwindigkeit der durch das Gehäuse strömenden Luft während bestimmter Messzyklen und/oder zeitlicher Zwischenräume zwischen solchen Messzyklen auf ein Maß eingestellt oder geregelt werden, dass zum einen eine ausreichende Kühlung der Maschine und zum anderen eine Art Basisdetektion von Leckagen möglich sind, bei der größere Leckagen mit ausreichend hoher Sicherheit erfasst werden.

Um auch kleine Leckagen sicher erfassen zu können, die bei einem eine ausreichende Kühlung der Maschine sicherstellenden Volumen- oder Massenstrom (also einem Volumen-/Massenstrom, der relativ hoch ist) nicht erfasst werden können, da der Anstieg der Feuchtigkeit der das Gehäuse durchströmenden Luftmenge für eine Erfassung zu gering wäre, kann ein Messzyklus eingestreut werden, bei dem der durch das Gehäuse geleitete Luftstrom sehr gering (geringer als der standardgemäß eingestellte Wert) oder so gering wie möglich eingestellt wird. Während eines solchen Messzyklus ist eine ausreichende Kühlung der Maschine unter Umständen nicht mehr vollständig gegeben, jedoch können auch kleine Leckagen so sicher erfasst werden, da sie aufgrund des geringen Volumen-/Massenstroms bzw. der geringen Strömungsgeschwindigkeit einen für eine Detektion ausreichenden oder deutlichen Anstieg der Feuchtigkeit des durch das Gehäuse geleiteten Luftstroms im Leckagefall bewirken.

Um kleinste Leckagen erfassen zu können, wird bei einer Ausführungsform der Erfindung der Luftstrom durch das Gehäuse für eine bestimmte Zeitdauer eingestellt, insbesondere vor einem Messzyklus, z.B. indem die Lüftungseinrichtung vollständig heruntergeregelt wird. Während dieser Zeitdauer, in der das Gehäuse nicht von Luft durchströmt wird, kommt es auch bei kleinsten Leckagen zu einem Ansteigen (Anreichern) der Feuchtigkeit der im Gehäuse vorhandenen Luftmenge, was bei einer nachfolgenden Messung einfach und sicher festgestellt werden kann. Insgesamt ermöglicht die Erfindung mit Vorteil, Leckagen nahezu unabhängig von der Menge und/oder des Massen- oder Volumenstroms der austretenden Dialysierflüssigkeit über einen breiten Bereich von Massen- bzw. Volumenströmen zu erfassen. Es ist möglich, Leckagen unterschiedlicher Größe zuverlässig und schnell zu detektieren, auch kleine und kleinste Leckagen. Dabei kann eine Kühlung der Maschine ohne wesentliche Beeinträchtigung betrieben werden.

Nach einer Ausführungsform der Erfindung werden die erfassten Werte der Parameter von Zu- und/oder Abluft genutzt, um die Kühlung und/oder Lautstärke der Maschine zu optimieren.

Nach einer weiteren Ausführungsform der Erfindung kann in dem Gehäuse ein Flies oder Ähnliches angeordnet sein oder werden. Dieses ist mit Vorteil geeignet und eingerichtet, ausgetretene Dialysierflüssigkeit vorzugsweise vollständig aufzunehmen und über seine große Oberfläche mit einer hohen Verdunstungsrate zeitnah abzugeben. Das Flies oder Ähnliches befindet sich vorzugsweise an solchen Stellen, an denen eine Leckage befürchtet wird. Nach einer weiteren Form der Erfindung können solche verdächtige Stellen bevorzugt überwacht werden, zum Beispiel indem der Luftstrom gezielt auf die Stelle der vermuteten Leckage gelenkt wird, beispielsweise mittels entsprechender Luftströmungsleiteinrichtungen oder mittels einer weiteren Lüfter- oder Ventilationseinheit.

Bei einer vorteilhaften Ausführungsform des Verfahrens der Erfindung kann eine Kalibrierung des Systems erfolgen, insbesondere vor Beginn einer Behandlung. Es kann dabei überprüft werden, ob sich Flüssigkeit im Inneren des Gehäuses der Dialysemaschine befindet. Dabei wird nach einem ersten Belüftungszylus des Gehäuses die Richtung der Durchströmung des Gehäuses umgekehrt. In dem Fall, dass im Inneren des Gehäuses keine Flüssigkeit vorhanden ist, ist ein Offset des zur Erfassung von Parametern der zu- bzw. abströmenden Luftmenge genutzten Sensors bei normaler Strömungsrichtung wie auch bei umgekehrter Strömungsrichtung durch das Gehäuse gleich groß. Falls Flüssigkeit, insbesondere Dialysierflüssigkeit, aufgrund einer undichten Stelle aus dem Dialysatsystem ausgetreten ist und im Inneren des Gehäuses vorliegt, unterscheiden sich die beiden bei der Kalibrierung gemessenen Offset-Werte. Der Sensor an der jeweiligen Abluftöffnung zeigt dabei den höheren Wert an. Bleibt der Offset bei beiden Strömungsrichtungen durch das Gehäuse konstant, kann er durch Kalibrierung des Sensors ausgeglichen werden. Mit besonderem Vorteil kann eine solche Überprüfung oder Kalibrierung mittels Umkehr der Strömungsrichtung der Luft durch das Gehäuse auch während einer Behandlung durchgeführt werden, ohne diese zu beeinträchtigen. Gemäß der Erfindung wird die Durchströmungsrichtung des Gehäuses wiederholt gewechselt, insbesondere regelmäßig gewechselt. Das kann parallel zum üblichen Behandlungsbetrieb erfolgen. Auf diese Weise kann eine Drift des Sensors/der Sensoren mehr oder weniger zeitnah erfasst und falls gewünscht korrigiert werden. Des Weiteren kann durch eine wiederholte Umkehr der Durchströmungsrichtung des Gehäuses eine Verschmutzung der Zu- bzw. Abluftöffnung vermindert werden. Schließlich kann durch eine Umkehr der Durchströmungsrichtung des Gehäuses 4 nach einem Feststellen von zusätzlicher Feuchtigkeit im Gehäuse eine Plausibilitätsprüfung durchgeführt werden. Dadurch kann festgestellt werden, ob ein Ansteigen eines Messwertes tatsächlich aufgrund von aus dem Dialysierflüssigkeitssystem in das Gehäuse gelangter Dialysierflüssigkeit verursacht wurde oder aber nur auf einem Messfehler oder steigender Luftfeuchtigkeit der Umgebungsluft beruht. Hierdurch kann mit Vorteil die Anzahl von Fehlalarmierungen verringert und die Verlässlichkeit des Systems bzw. des Verfahrens verbessert werden.

Die Erfindung wird im Folgenden anhand beispielhafter, nicht einschränkender und in den angehängten Figuren gezeigter Ausführungsformen näher erläutert. Dabei zeigt:
Figur 1 eine erste Ausführungsform einer Dialysemaschine nach der Erfindung,
Figur 2 eine zweite Ausführungsform einer Dialysemaschine nach der Erfindung und
Figur 3 einen Signalverlauf für Feuchtewerte bei Messungen bei der Vorrichtung nach Figur 1 bzw. 2.

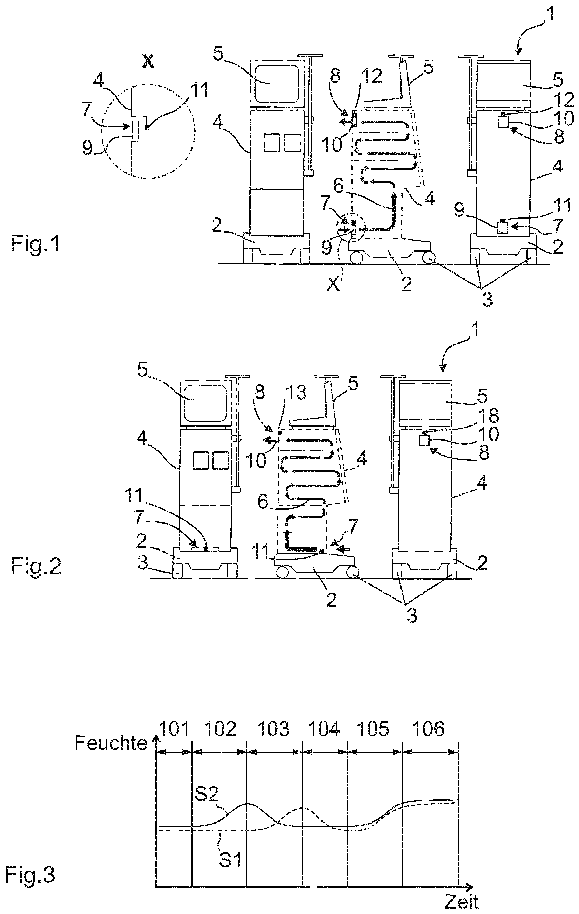

Figur 1 zeigt eine erfindungsgemäße Dialysemaschine 1 in einer Vorderansicht, einer Rückansicht und einer teilgeschnittenen Seitenansicht. Sie besitzt vorzugsweise einen fahrbaren Fuß 2 mit Rollen 3. Auf diesem ist ein gegenüber der Umgebung hermetisch dichtes (fluiddichtes) Gehäuse 4 angeordnet, welches wiederum eine Anzeige- und Bedieneinheit 5 trägt. In dem hermetisch dichten Gehäuse 4 ist wenigstens ein in der Figur nicht näher dargestelltes Dialysierflüssigkeitssystem angeordnet/untergebracht. Dieses enthält/führt eine Dialysierflüssigkeit, die mittels des Dialysierflüssigkeitssystems in bekannter Weise auf- und verarbeitet wird.

Das Gehäuse 4 wird unter anderem zu Kühlzwecken belüftet, indem ein Luftstrom durch eine Zuluftöffnung 7 in die Maschine 1, durch das Gehäuse 4 und über eine hiervon separate Abluftöffnung 8 aus der Maschine 1 herausgeführt wird. In der mittleren Darstellung der Figur 1 ist der Strömungsweg 6 durch das Gehäuse 4 schematisch mittels mäanderförmiger Pfeile angedeutet. Der Strömungsweg 6 ist derart ausgebildet, dass der durch das Gehäuse 4 geleitete Luftstrom möglichst sämtliche Bereiche des Gehäuses 4 durchströmt. Er kann insbesondere so gestaltet sein, dass Luft gezielt in Bereiche, in denen ein Auftreten von Leckagen vermutet wird, oder in Bereiche, in denen sich Leckageflüssigkeit bevorzugt sammelt, geführt wird.

In der Zuluftöffnung 7 ist ein erster Lüfter 9 angeordnet. In der Abluftöffnung 8 ist ein zweiter Lüfter 10 angeordnet. Bei der dargestellten Strömungsrichtung der Luft durch das Gehäuse 4 (Strömungsweg 6) wird der Luftstrom vorzugsweise mittels des zweiten Lüfters 10 bewirkt, der Luft aus dem Gehäuse 4 herausfördert. Der erste Lüfter 9 wird dabei nicht oder mit einer geringeren Förderleistung als der erste Lüfter 10 angetrieben, so dass im Gehäuse 4 gegenüber der Umgebung ein Unterdruck entsteht. Auf diese Weise ist sichergestellt, dass Luft nur über den zweiten Lüfter 10 und die Abluftöffnung 8 aus dem Gehäuse gelangen und nur solche zusätzliche Feuchtigkeit in dem Luftstrom aufgrund von Leckage im Dialysierflüssigkeitssystem sicher erfasst werden kann.

Die Erfassung der Feuchtigkeit der einströmenden Luft wird bei dem gezeigten Strömungsweg 6 mittels eines ersten Feuchtigkeitssensors 11 bewirkt, der in der oder angrenzend an die Zuluftöffnung 7 angeordnet ist. Die Feuchtigkeit des die Maschine 1 verlassenden Abluftstroms wird mittels eines zweiten Feuchtigkeitssensors 12 erfasst, der in der oder an die Abluftöffnung 8 angrenzend angeordnet ist. Durch einen Vergleich der durch die beiden Sensoren 11,12 ermittelten Feuchtigkeitswerte können Rückschlüsse auf zusätzlich in das gegenüber der Umgebung hermetisch dichten Gehäuses 4 eingebrachte Feuchtigkeit, zum Beispiel aufgrund von Leckage des Dialysierflüssigkeitssystems, gezogen werden, wie im Nachfolgenden genauer beschrieben wird.

Die in der Figur 1 dargestellte Dialysemaschine kann in umgekehrter Richtung wie dargestellt belüftet werden. Bei Umkehr der Durchströmungsrichtung 6 strömt Luft über die Abluftöffnung 8 in das Gehäuse 4 ein und verlässt es wieder über die Zuluftöffnung 7. In diesem Fall wird die Belüftung vorzugsweise mittels des ersten Lüfters 9 bewirkt, so dass im Gehäuse 4 - wie vorstehend beschrieben - ein Unterdruck gegenüber der Umgebung herrscht.

Die Figur 2 zeigt eine zweite Ausführungsform der Dialysemaschine 1 in einer Vorderansicht, einer Rückansicht und einer teilgeschnittenen Seitenansicht. Da diese in den wesentlichen Grundzügen der Ausführungsform der Figur 1 gleicht, wird auf die vorstehende Beschreibung dieser Ausführungsform verwiesen und nachfolgend im Wesentlichen nur Abweichungen davon beschrieben.

Das Gehäuse 4 weist ebenfalls eine Zuluftöffnung 7 und eine Abluftöffnung 8 auf. In der Abluftöffnung 8 ist ein Lüfter 13 als einziger Lüfter dieser Ausführungsform angeordnet. In der oder angrenzend an die Zuluftöffnung 7 ist ein erster Feuchtigkeitssensor 11 angeordnet. In der oder angrenzend an die Abluftöffnung 8 ist schließlich ein zweiter Feuchtigkeitssensor 12 angeordnet. Mittels der Feuchtigkeitssensoren 11,12 wird die Feuchtigkeit des die Zuluftöffnung 7 bzw. die Abluftöffnung 8 durchströmenden Luftstroms gemessen.

Die Durchströmung der in der Figur 2 dargestellten Ausführungsform erfolgt im Normalfall wie mittels der Pfeile zum Durchströmungsweg 6 angedeutet. Der Lüfter 13 arbeitet dabei im Saugbetrieb und entlüftet das Gehäuse 4 durch Absaugen von Luft aus dem Gehäuseinnenraum in die Umgebung. Sie kann jedoch auch in umgekehrter Richtung wie dargestellt belüftet werden. Dazu wird der Lüfter 13 im Blasbetrieb betrieben, wobei er Luft aus der Umgebung in das Gehäuse 4 hineinfördert.

Figur 3 zeigt den theoretischen Signalverlauf bei einer Messung der Feuchtigkeitswerte mittels der beiden Feuchtigkeitssensoren 11,12 der Ausführungsformen der Figuren 1 und 2. Die gestrichelte Kurve S1 stellt den Signalverlauf des ersten Feuchtigkeitssensors 11 dar, die durchgezogene Kurve S2 den Signalverlauf des zweiten Feuchtigkeitssensors 12. Auf der Abszisse des Diagramms der Figur 3 sind die mittels der Feuchtigkeitssensoren gemessenen Feuchtigkeitswerte aufgetragen. Auf der Ordinate ist die Zeit aufgetragen.

In einem ersten Zeitabschnitt 101 erfolgt eine Kalibrierung der beiden Feuchtigkeitssensoren 11 und 12. Während eines nachfolgenden Zeitabschnitts 102 tritt eine Leckage im Dialysierflüssigkeitssystem der Maschine 1 auf. Aus diesem austretende (leckende) Dialysierflüssigkeit gelangt in das gegenüber der Umgebung hermetisch dichte Gehäuse 4, verdunstet dort und führt zu einem Anstieg der Luftfeuchtigkeit innerhalb des Gehäuses 4 sowie gegenüber der Umgebungsluft, der aufgrund der Strömungsrichtung im Gehäuse 4 nur vom zweiten Feuchtigkeitssensor 12, der in der Abluftöffnung 8 angeordnet ist, erfasst werden kann. Der erste Feuchtigkeitssensor 11 in der Zuluftöffnung erfasst im Zeitabschnitt 102 fortwährend die (in der Regel konstante) Feuchtigkeit der in das Gehäuse 4 einströmenden Umgebungsluft. Dies spiegelt sich deutlich im Verlauf der Signalkurven S1 und S2 wieder: S1 bleibt im Wesentlichen konstant, während S2 im Verlauf des Zeitabschnitts 102 (deutlich) ansteigt.

In einem nachfolgenden Zeitabschnitt 103, nach Detektion des Anstiegs des Signals S2 des zweiten Sensors 12, erfolgt eine Plausibilitätsüberprüfung, indem die Durchströmungsrichtung des Gehäuses 4 wie vorstehend mit Bezug auf die Figuren 1 bzw. 2 beschrieben, umgekehrt wird. Der zweite Sensor 12 wird dann nicht mehr mit Luft aus dem Gehäuse 4 beaufschlagt, sondern mit Umgebungsluft (von in der Regel konstanter Luftfeuchtigkeit). Sein Signal S2 sinkt daher im Verlauf des Zeitabschnitts 103 wieder auf den Normalwert der Umgebungsluft ab. Der erste Sensor 11 hingegen wird im Zeitabschnitt 103 mit aus dem Gehäuse 4 ausströmender Abluft beaufschlagt. Im Falle einer Leckage im Dialysierflüssigkeitssystem, die zu einem Eindringen von Dialysierflüssigkeit in das Gehäuse 4 führt, stellt der Sensor 11 ein Ansteigen der Luftfeuchtigkeit innerhalb des Gehäuses 4 fest. Sollte das Ansteigen des Signals S2 des zweiten Sensors während des Zeitabschnitts 102 hingegen auf einem Sensorfehler oder Ähnlichem beruhen, also keine Dialysierflüssigkeit in das Gehäuse 4 eingetreten sein, würde der Sensor 11 während des Zeitabschnitts 103 keine erhöhte Feuchtigkeit im Gehäuse 4 erfassen und sein Signal S1 bliebe konstant. Eine erste Anzeige auf eine Leckage kann daher einfach überprüft werden, wodurch Fehlalarmierungen verringert oder verhindert werden können und die Verlässlichkeit des Systems steigt.

In anderen Worten ausgedrückt werden die beiden Sensoren 11, 12 an den separaten Belüftungsöffnungen abwechselnd als Einlass- und Auslass-Sensor betrieben, um so mögliche Messfehler auszuschließen oder zu verringern.

Liegt eine Leckage vor - und dieser Fall ist in Figur 3 dargestellt - wird das Leck beseitigt. Dies erfolgt zu Beginn eines weiteren Zeitabschnitts 104. Aufgrund der fortgesetzten Belüftung des Gehäuses 4 im Zeitabschnitt 104 sinkt die Luftfeuchtigkeit und damit das Signal S1 langsam ab. Bei einem weiteren störungsfreien Verlauf der Therapie bleiben die Signale S1 und S2 dann konstant, was in der Figur 3 nur kurz angedeutet ist.

Figur 3 zeigt einen weiteren Zeitabschnitt 105, in dem Feuchtigkeit von außerhalb eindringt oder eine externe Leckage außerhalb des Gehäuses 4 auftritt. In diesen Fällen stellen beide Sensoren 11 und 12 ein Ansteigen der Feuchtigkeit fest und beide Signale S1 und S2 steigen an. Aufgrund des zeitgleichen Ansteigens beider Signale S1 und S2 kann eine interne Leckage, also ein Austreten von Dialysierflüssigkeit aus dem Dialysierflüssigkeitssystem in das Gehäuse 4, sicher ausgeschlossen werden. Bei einem nachfolgenden Plausibilitätscheck im Zeitraum 106 wird die Maschine - wie bereits zuvor mit Bezug auf den Zeitraum 103 beschrieben - mit umgekehrter Durchströmungsrichtung betrieben, wobei im Falle einer externen Leckage die Sensorwerte S1 und S2 unverändert bleiben.

Abschließend sei darauf hingewiesen, dass anstelle der vorstehend beschriebenen Feuchtigkeitssensoren zur unmittelbaren Erfassung des Flüssigkeitsanteils in der Luft auch noch weitere oder andere Parameter der Luft sensorisch erfasst werden können, welche Rückschlüsse auf die Luftfeuchtigkeit innerhalb des Gehäuses 4 gegenüber der Umgebungsluft ziehen lassen. Beispielsweise kann die Temperatur gemessen werden, welche bei einem normalen Betrieb der Maschine im Wesentlichen konstant ist, sich jedoch bei einem eintretenden Luftfeuchtigkeitsanstieg ändert. Auch können mehrere gleiche oder unterschiedliche Sensoren an den Belüftungsöffnungen und/oder innerhalb des Gehäuses 4 angeordnet sein, um ggf. sogar Hinweise auf Leckageorte zu erhalten.

Schließlich reicht es im einfachsten Fall aus, nur an der Auslassöffnung einen Feuchtigkeitssensor anzubringen. In diesem Fall könnte über diesen Sensor der Feuchtigkeitsgehalt der Umgebungsluft vor Beginn einer Behandlung gemessen und gespeichert werden, da sich dieser Feuchtigkeitswert nicht oder nur sehr langsam ändert. Mit Beginn der Behandlung misst dann der einzige Sensor den Feuchtigkeitsanteil der Ausblasluft, worauf der aktuelle gemessene Wert mit dem vorab gespeicherten Wert verglichen wird.

Zusammenfassend wird eine Dialysemaschine mit Leckageerkennung sowie ein Verfahren zur Erkennung von Leckagen im Dialysierflüssigkeitskreis der extrakorporalen Blutbehandlungsmaschine offenbart, wobei wenigstens ein Teil des hinsichtlich Leckage zu überwachenden Dialysierflüssigkeitssystems in einem hermetisch gegenüber der Umgebung dichten Gehäuse aufgenommen ist und das Gehäuse kontrolliert belüftet wird bzw. belüftbar ist, und wobei ein Parameter, vorzugsweise Luftfeuchtigkeit der in das Gehäuse einströmenden Luft mit einem entsprechenden Parameter, vorzugsweise Luftfeuchtigkeit der aus dem Gehäuse ausströmenden Luft verglichen wird, um aus einer möglicher Weise auftretenden Differenz auf das Vorhandensein einer Leckage zu schließen.

## Patentansprüche

1. Dialysemaschine (1) mit einem Gehäuse (4), das wenigstens eine erste Belüftungsöffnung (7) und wenigstens eine zweite Belüftungsöffnung (8) aufweist, wobei das Gehäuse (4) mit Ausnahme der Belüftungsöffnungen (7,8) gegenüber der Umgebung hermetisch dicht ausgebildet ist,
wobei in dem Gehäuse (4) wenigstens ein hinsichtlich Leckage zu erfassender oder zu überwachender Teil eines Dialysierflüssigkeitssystems untergebracht ist,
wobei wenigstens eine Lüftungseinrichtung (9,10) vorgesehen ist, mittels der Umgebungsluft von der ersten oder der zweiten Belüftungsöffnung (7,8) durch das Gehäuse (4) zur anderen Belüftungsöffnung (8 bzw. 7) förderbar ist bzw. gefördert wird, und
wobei die Dialysemaschine (1) des Weiteren wenigstens eine Messeinrichtung (11,12,13) aufweist, mittels der die Feuchtigkeit der über die Belüftungsöffnungen (7,8) in das Gehäuse ein- und/oder aus dem Gehäuse ausströmenden Luft bestimmbar ist bzw. bestimmt wird, charakterisiert dadurch, dass die wenigstens eine Lüftungseinrichtung über eine Steuerung zeitlich versetzt in zwei Förderrichtungen von entgegengesetzter Richtung betreibbar ist.

2. Dialysemaschine nach Anspruch 1, wobei der Strömungsweg der durch das Gehäuse (4) geförderten Luft von der ersten Belüftungsöffnung (7) durch das Gehäuse (4) zur zweiten Belüftungsöffnung (8) verläuft, und in der oder angrenzend an die erste Belüftungsöffnung (7) eine erste Messeinrichtung und in der oder angrenzend an die zweite Belüftungsöffnung (8) eine zweite Messeinrichtung angeordnet sind.

3. Dialysemaschine nach einem der vorstehenden Ansprüche, wobei die Messeinrichtung ein Feuchtigkeitssensor ist.

4. Dialysemaschine nach einem der vorstehenden Ansprüche, wobei in dem Gehäuse (4) wenigstens eine weitere Lüftungseinrichtung angeordnet ist zur gezielten Lenkung und Verteilung der das Gehäuse (4) durchströmenden Luft, insbesondere zu Stellen, an denen sich im Falle einer Leckage aus dem Dialysierflüssigkeitssystem ausgetretene Flüssigkeit bevorzugt sammelt.

5. Dialysemaschine nach einem der vorstehenden Ansprüche, wobei in dem Gehäuse (4) ein Vlies angeordnet ist, vorzugsweise im Bereich von leckagegefährdeten Stellen.

6. Verfahren zum Erfassen von Leckage in einem Dialysierflüssigkeitssystem einer Dialysemaschine (1), bei dem ein Gehäuse (4), das eine erste Belüftungsöffnung (7) und eine zweite Belüftungsöffnung (8) aufweist und im Übrigen gegenüber der Umgebung hermetisch dicht ist, wenigstens einen hinsichtlich Leckage zu erfassenden Teil des Dialysierflüssigkeitssystems enthält,
wobei das Gehäuse (4) über die Belüftungsöffnungen (7,8) belüftet wird, wobei Zustandsparameter, vorzugsweise Luftmasse, Temperatur und/oder Feuchtigkeit, von dem Gehäuse (4) zugeführter Zuluft sowie von aus dem Gehäuse (4) abgeführter Abluft erfasst und verglichen werden und aus der Differenz dieser Werte ein Feuchtigkeitseintrag in das Gehäuse (4) bestimmt wird, der nicht aus der Umgebungsluft stammt, charakterisiert dadurch, dass die Durchströmungsrichtung des Gehäuses wiederholt, insbesondere regelmässig, gewechselt wird.

7. Verfahren nach Anspruch 6, wobei Zustandsparameter der in das Gehäuse (4) einströmenden Zuluft und/oder der aus dem Gehäuse (4) herausströmenden Abluft in diskreten Messzyklen erfasst werden.

8. Verfahren nach Anspruch 6 oder 7, wobei im Falle einer Erfassung eines eine Leckage anzeigenden Zustandsparameters eine Plausibilitätsprüfung durchgeführt wird, indem die Strömungsrichtung der Luft durch das Gehäuse (4) umgekehrt wird und ein dabei erfasster Zustandsparameter mit dem eine Leckage anzeigenden Zustandsparameter bei vorheriger Strömungsrichtung verglichen wird.

9. Verfahren nach einem der vorstehenden Ansprüche 6 bis 8, wobei die Belüftung des Gehäuses (4) für einen Zeitabschnitt, insbesondere vor einem Messzyklus, eingestellt oder abgesenkt wird und/oder eine nachfolgende Messung mit einer möglichst geringen Strömungsrate durchgeführt wird, insbesondere mit einer Strömungsrate, die für eine ausreichende Kühlung der Maschine nicht genügt.

10. Verfahren nach einem der vorstehenden Ansprüche 6 bis 9, wobei insbesondere vor einer Therapie eine Kalibrierung durchgeführt wird, indem Luft in einer ersten Strömungsrichtung durch das Gehäuse (4) geleitet und ein Messwert von einer Messeinrichtung erfasst wird, und nachfolgend Luft mit umgekehrter Strömungsrichtung durch das Gehäuse (4) geleitet und der Messwert von der Messeinrichtung erfasst und mit dem zuvor erfassten Messwert verglichen wird.

11. Verfahren nach Anspruch 10, wobei die Differenz der erfassten Messwerte gespeichert und als Korrekturfaktor für nachfolgende Messungen verwendet wird.

## Claims

1. Dialysis machine (1) comprising a housing (4) having at least one first ventilation aperture (7) and at least one second ventilation aperture (8),
wherein the housing (4) with the exception of the ventilation apertures (7, 8) is configured to be hermetically sealed against the ambience,
wherein at least part of a dialysis fluid system to be detected or to be monitored in terms of leakage is accommodated in the housing (4),
wherein at least one venting means (9, 10) is provided by means of which ambient air can be or is conveyed from the first or second ventilation aperture (7, 8) through the housing (4) to the other ventilation aperture (8 or 7), and
wherein the dialysis machine (1) moreover is provided with or includes at least one measuring means (11, 12, 13) by means of which the humidity of the air flowing into and/or out of the housing via the ventilation apertures (7, 8) can be or is determined, **characterized in that** the at least one venting means is operable via a control at staggered intervals in two oppositely directed conveying direction.

2. Dialysis machine according to claim 1, wherein the flow path of the air conveyed through the housing (4) extends from the first ventilation aperture (7) through the housing (4) to the second ventilation aperture (8) and in or adjacent to the first ventilation aperture (7) a first measuring means is arranged and in or adjacent to the second ventilation aperture (8) a second measuring means is arranged.

3. Dialysis machine according to any one of the preceding claims, wherein the measuring means is a humidity sensor.

4. Dialysis machine according to any one of the preceding claims, wherein in the housing (4) at least one further venting means is arranged for specifically directing and distributing the air flowing through the housing (4), especially to positions where fluid escaped from the dialysis fluid system in the case of leakage preferably accumulates.

5. Dialysis machine according to any one of the preceding claims, wherein a nonwoven is arranged in the housing (4), preferably in the area of positions susceptible to leakage.

6. A method of detecting leakage in a dialysis fluid system of a dialysis machine (1), in which a housing (4) including a first ventilation aperture (7) and a second ventilation aperture (8) and otherwise being hermetically sealed against the ambience contains at least part of the dialysis fluid system to be detected in terms of leakage,
wherein the housing (4) is ventilated via the ventilation apertures (7, 8), wherein parameters of state, preferably air mass, temperature and/or humidity, of inlet air supplied to the housing (4) as well as of outlet air discharged from the housing (4) are detected and compared and from the difference of these values a humidity input to the housing (4) is determined which does not originate from the ambient air, **characterized in that** the direction of flow through the housing is repeatedly alternated, especially regularly alternated.

7. The method according to claim 6, wherein parameters of state of the inlet air flowing into the housing (4) and/or the outlet air flowing out of the housing (4) are detected in discrete measuring cycles.

8. The method according to claim 6 or 7, wherein, when a parameter of state indicative of leakage is detected, a plausibility check is performed by reversing the flow direction of the air through the housing (4) and comparing a thus detected parameter of state to the parameter of state indicative of leakage for the preceding flow direction.

9. The method according to any one of the preceding claims 6 to 8, wherein the ventilation of the housing (4) is stopped or reduced for a time interval, especially before a measuring cycle, and/or a subsequent measurement is performed at a flow rate being as low as possible, especially at a flow rate that does not suffice for sufficient cooling of the machine.

10. The method according to any one of the preceding claims 6 to 9, wherein especially before a therapy a calibration is performed by passing air in a first flow direction through the housing (4) and a measuring value being detected by the measuring means, and after that air having a reversed direction of flow is passed through the housing (4) and is compared to the afore-detected measuring value.

11. The method according to claim 10, wherein the difference of the measuring values detected is stored and used as correction factor for subsequent measurements.

## Revendications

1. Machine de dialyse (1) comprenant un boîtier (4), qui présente au moins une première ouverture d'aération (7) et au moins une deuxième ouverture d'aération (8), dans laquelle le boîtier (4) est réalisé de manière hermétiquement étanche par rapport à l'environnement à l'exception des ouvertures d'aération (7, 8),
dans laquelle au moins une partie, à détecter ou à surveiller en matière de fuite, d'un système de liquide de dialyse est abritée dans le boîtier (4),
dans laquelle au moins un dispositif de ventilation (9, 10) est prévu, au moyen duquel de l'air environnant peut être refoulé ou est refoulé de la première ou de la deuxième ouverture d'aération (7, 8), à travers le boîtier (4), en direction de l'autre ouverture d'aération (8 ou 7), et
dans laquelle la machine de dialyse (1) présente par ailleurs au moins un dispositif de mesure (11, 12, 13), au moyen duquel l'humidité du flux d'air entant dans le boîtier et/ou sortant du boîtier par l'intermédiaire des ouvertures d'aération (7, 8) peut être définie ou est définie, **caractérisée en ce que** l'au moins un dispositif de ventilation peut fonctionner par l'intermédiaire d'une commande avec un décalage dans le temps dans deux directions de refoulement de sens opposé.

2. Machine de dialyse selon la revendication 1,
dans laquelle le trajet d'écoulement de l'air refoulé à travers le boîtier (4) s'étend de la première ouverture d'aération (7), à travers le boîtier (4), en direction de la deuxième ouverture d'aération (8), et un premier dispositif de mesure est disposé dans la première ouverture d'aération (7) ou de manière contiguë à cette dernière et un deuxième dispositif de mesure est disposé dans la deuxième ouverture d'aération (8) ou de manière contiguë à cette dernière.

3. Machine de dialyse selon l'une quelconque des revendications précédentes, dans laquelle le dispositif de mesure est un capteur d'humidité.

4. Machine de dialyse selon l'une quelconque des revendications précédentes, dans laquelle au moins un autre dispositif de ventilation est disposé dans le boîtier (4) afin de diriger et de répartir de manière ciblée le flux d'air traversant le boîtier (4), en particulier vers des emplacements, au niveau desquels en cas de fuite, le liquide sortant du système de liquide de dialyse s'accumule de manière préférée.

5. Machine de dialyse selon l'une quelconque des revendications précédentes, dans laquelle un non-tissé est disposé dans le boîtier (4), de préférence dans la zone d'emplacements menacés par des fuites.

6. Procédé servant à détecter une fuite dans un système de liquide de dialyse d'une machine de dialyse (1), dans le cadre duquel un boîtier (4), qui présente une première ouverture d'aération (7) et une deuxième ouverture d'aération (8) et qui par ailleurs est hermétiquement étanche par rapport à l'environnement, contient au moins une partie, à détecter en matière de fuite, du système de liquide de dialyse,
dans lequel le boîtier (4) est aéré par l'intermédiaire des ouvertures d'aération (7, 8), dans lequel des paramètres d'état, de préférence la masse d'air, la température et/ou l'humidité, de l'air amené par le boîtier (4) ainsi que de l'air vicié évacué du boîtier (4) sont détectés et comparés et un apport d'humidité est défini dans le boîtier (4) à partir de la différence desdites valeurs, lequel apport d'humidité ne provient pas de l'air environnant, **caractérisé en ce que** la direction d'écoulement traversant du boîtier est changée de manière répétée, en particulier de manière régulière.

7. Procédé selon la revendication 6, dans lequel des paramètres d'état du flux d'air entrant dans le boîtier (4) et/ou du flux d'air vicié sortant du boîtier (4) sont détectés dans des cycles de mesure discrets.

8. Procédé selon la revendication 6 ou 7, dans lequel dans le cas d'une détection d'un paramètre d'état indiquant une fuite, un contrôle de plausibilité est effectué en ce que la direction d'écoulement de l'air à travers le boîtier (4) est inversée et un paramètre d'état détecté dans ce cadre est comparé au paramètre d'état indiquant une fuite dans le cas de la direction d'écoulement précédente.

9. Procédé selon l'une quelconque des revendications précédentes 6 à 8, dans lequel l'aération du boîtier (4) est réglée ou abaissée pour un laps de temps, en particulier avant un cycle de mesure, et/ou une mesure subséquente.est effectuée à un taux d'écoulement dans la mesure du possible faible, en particulier à un taux d'écoulement, qui ne suffit pas pour un refroidissement suffisant de la machine.

10. Procédé selon l'une quelconque des revendications précédentes 6 à 9, dans lequel un étalonnage est effectué en particulier avec un traitement, en ce que l'air est acheminé à travers le boîtier (4) dans une première direction d'écoulement et une valeur de mesure est détectée par un dispositif de mesure, puis l'air est acheminé à travers le boîtier (4) selon une direction d'écoulement inversée et la valeur de mesure est détectée par le dispositif de mesure et est comparée à la valeur de mesure détectée au préalable.

11. Procédé selon la revendication 10, dans lequel la différence des valeurs de mesure détectées est mémorisée et est utilisée en tant que facteur de correction pour des mesures subséquentes.
